# EUROPEAN PATENT SPECIFICATION

(11) **EP 3 280 474 B1**
(45) Date of publication and mention of the grant of the patent: **04.11.2020**
(21) Application number: 16776979.3
(22) Date of filing: 08.01.2016
(51) Int. Cl.: A61M 11/00, A61M 15/00, A61M 15/08, B05B 17/00

(54) **A NASAL MEDICATION DELIVERY DEVICE**
VORRICHTUNG FÜR NASALE MEDIKAMENTENVERABREICHUNG
DISPOSITIF D'ADMINISTRATION D'UN MÉDICAMENT NASAL

(30) Priority: 09.04.2015 NZ 70686415
(43) Date of publication of application: 14.02.2018
(73) Proprietor: AFT Pharmaceuticals Limited, Auckland 0622 (NZ)
(72) Inventor: ATKINSON, Hartley Campbell, Takapuna, Auckland 0632 (NZ); WOODHEAD, Brendon John, Greeenhithe, Auckland (NZ)
(74) Representative: Bittner, Thomas L.
(86) International application number: PCT/NZ2016/050002
(87) International publication number: WO 2016/163895

(56) References cited:
- EP-A1- 2 021 131
- WO-A1-2004/017848
- WO-A1-2004/045690
- WO-A1-2006/125251
- WO-A2-2013/132427
- WO-A2-2014/165694
- US-A1- 2006 243 274
- US-A1- 2008 156 320
- US-A1- 2011 120 456
- US-A1- 2012 118 283
- US-A1- 2013 112 197
- US-A1- 2014 352 689
- US-B2- 7 347 201

## Description

### FIELD OF INVENTION

This invention relates to a device for delivering medication to the nasal cavity of a human patient.

### BACKGROUND

Devices which deliver medication to the nasal cavities are known. In some cases they create an aerosol from liquid medication using compressed gas or piezoelectric energy. They are often referred to as "nebulisers" and they typically have a chamber in which the medication is energised. The medicament is then caused to contact a mesh, or to move through a jet nozzle, to enter the nasal cavities as an aerosol. The mesh may be vibrated directly via a piezo-oscillating element, or indirectly as a result of acoustic energy. The acoustic energy may also be used to drive the medication to the mesh.

Some nebulisers have a drawback in that their delivery mechanism requires an undesirably large minimum volume of medication before they can do their work. When the medication falls to below that volume a relatively large residual amount is wasted. It is an object of a preferred embodiment of the present invention to go at least some way towards alleviating this problem. However this and any other objects of the preferred embodiment should not be taken as a limitation on the scope of the claims. The object of the invention in its broadest form is simply to provide a useful choice.

The prior art includes the following solutions. European patent No. EP 2021131 B1 relates to an atomisation apparatus and to an atomiser for nebulizing, liquid treatment and/or filtration devices, and claims an atomisation apparatus comprising: a container being adapted to hold a liquid to be atomized, part of the container forming an ultrasonic transducer; an electric generator being operatively coupled to the ultrasonic transducer and arranged to cause said ultrasonic transducer to oscillate; and a mesh; wherein the ultrasonic transducer is concave to transmit energy to the liquid at a focal zone, and wherein the mesh is disposed adjacent the container at the focal zone for contact with the liquid which at least in part passes through the mesh and is atomized.

Patent application No. WO2014/165694 A2 is directed to methods and apparatuses for intranasal delivery of a substance to a subject, and claims a nasal delivery device for delivering a treatment agent from a dose container to a subject, the device comprising: a handpiece that can be held by a user and having an activation member for triggering the delivery of the treatment agent to the subject; an intranasal portion extending from the handpiece and having a dose container receiving area and an aerosolizing member, the aerosolizing member being configured to aerosolize the treatment agent and eject an aerosolized plume containing the treatment agent into a nare of the subject. In embodiments, the nasal delivery device of this document has the following features: the intranasal portion has a distal end that extends into a nare of the subject, further comprising a vibrating-mesh nebulizer located at the distal end of the intranasal portion, wherein the vibrating-mesh nebulizer is angled relative to an axis of the intranasal portion to eject the aerosolized plume through a nasal valve of the subject; the device further comprises an ultrasonic actuator at least partially located in the handpiece, wherein the ultrasonic actuator comprises a long horn actuator that comprises a first portion having a piezoelectric stack within the handpiece and a second portion that comprises a coupling tip that extends into the intranasal portion; the device further comprises a dose container, the dose container being sized to be received on or within the intransal portion, wherein the dose container extends over the intransal portion to restrict direct contact between the intranasal portion and the subject, the device further comprising a pressurizing feed system for delivering treatment agent from the dose container at a controlled rate. Patent application No. US 2012/118283 A1 relates generally to the delivery of agents, and more particularly, to systems and methods for delivery of agents using portable aerosol devices. This document claims a system for administering an agent, comprising: a housing; an air source operable to provide positive pressure air; a nebulizer operable for receiving an agent from a vial coupled to the housing and converting the agent into an aerosolized agent; a mixing chamber to receive the aerosolized agent from the nebulizer and air from the air source; a release mechanism operable for releasing a quantity of the agent from the vial to the nebulizer, wherein the agent is converted into an aerosolized agent that is mixed with air from the air source in the mixing chamber; a prong with an inlet and an outlet, wherein the inlet is operable to receive the aerosolized agent and air mixture from the mixing chamber, and the outlet is operable to deliver the aerosolized agent and air mixture to a patient when the prong is inserted into a patient's orifice; and an anti-backflow valve between the mixing chamber and the prong outlet, operable to permit flow from the mixing chamber to the prong, and to inhibit flow in a reverse direction. In embodiments, the system of this document has the following features: said nebulizer comprises an ultrasonic nebulizer, wherein said ultrasonic nebulizer comprises a piezoelectric actuator operatively connected to a power source.

US patent application No. US 2011/120456 A1 describes an aerosol therapy device, wherein an aerosol is generated in an aerosol generating device and is supplied through a nosepiece to a patient's nasal cavities via a single pulsed main aerosol flow.

Patent application No. US 2013/112197 A1 relates to a method for operating an aerosol delivery device (nebuliser) and an aerosol delivery device implementing this method. In an embodiment, this document discloses an aerosol delivery device which contains an aerosol generator, which may be an inhaler, atomiser or nebuliser, especially a nebuliser operating with a vibrating membrane or pores of a defined size. The aerosol delivery device comprises a connector for connection with a gas compressor as a source of compressed air and an adaptation element that is equipped with a nosepiece for adaptation to (communication with) a patient's nasal cavity. A fluid container for receiving a fluid to be nebulised is disposed between the connector and the adaptation element. A gas compressor is used as the gas conveying element and a sinus wave generator that is also connected to the connector is optionally used as a pulsator in a combined mode. The pulsator and the gas compressor together form a gas supply unit (air supply unit). One end of the fluid container can be securely and tightly closed with a screw cap. At its other end, opposite the screw cap, the fluid container may have a tapered portion that tapers towards a fluid chamber. The fluid chamber may be sealed by a sealing lip that forms a part of the chamber and is tightly pressed against a membrane. The membrane is provided with a plurality of minute openings or holes with diameters in the micrometer range that fully penetrate the membrane. Furthermore, the membrane can be vibrated (or oscillated), for example with the use of a piezoelectric element, such that the direction of the vibrations is perpendicular to the plane of the membrane. By inducing such vibrations in the membrane, fluid contained in the fluid chamber is passed through the minute openings of the membrane and nebulised into the nebuliser chamber formed at the other side (opposite the fluid chamber) of the membrane. In this way, the fluid chamber and the membrane together form a vibrating membrane nebuliser device (aerosol generator). A detailed description of this common concept is given, for example, in U.S. Patent 5,518,179. A control comprises a computer and a first control element, such as a transistor, that is connected to the membrane for stopping the membrane vibration and hence the aerosol generation before an optional step of pulsating (vibrating) the aerosol may be carried out.

US patent application No. US 2006/243274 A1 discloses a drug delivery device that uses an aerosol generator to nebulize a drug solution.

Patent application No. WO 2004/017848 A1 describes an ultrasonic nebulizer having a bowl-shaped container and a tubular energy transmitter in the form of acoustic transmitter pipe for delivering a substance in an aerosol form into a cellular organism.

### SUMMARY OF THE INVENTION

A delivery device for nasal medication according to claim 1 is provided.

Optionally the device has a mouthpiece adapted for a human user to blow into and sensing means which, in response to such blowing, causes the medication to be energised and the aerosol to form.

Optionally the energising chamber has a volume of 0.1 cm3 to 0.8 cm3, and preferably a volume of 0.2 cm³ to 0.4 cm³.

Optionally the device is formed such that it causes the mesh to vibrate as the medication contacts it so as to assist in creating the aerosol.

Optionally the mesh is integral with the prong.

Optionally the mesh is immediately adjacent to the exit so as to prevent or minimise condensation of the aerosol inside the device.

Optionally the energising chamber, the generator and exit are at an angle of about 40° to about 80°, and preferably about 50° to about 70°, with respect to the mouthpiece.

Optionally the medication is a pharmaceutical or saline solution prior to being energised.

Optionally the feeder chamber has a funnel or flume formed to naturally encourage the medication into the energising chamber.

Optionally the energising chamber has a tapered floor to urge medication to gather at the focal zone.

Optionally the energising chamber has a weil to urge medication to gather at the focal zone.

Optionally the energising chamber is symmetrical.

Optionally the energising chamber is asymmetrical.

Optionally the feeder chamber plus the energising chamber collectively have sufficient medication for:
- only 5 or fewer patient doses;
- only 4 or fewer patient doses;
- only 3 or fewer patient doses;
- only 2 or fewer patient doses; or
- only 1 patient dose;
(a dose in this context is the amount of medication per administration event, as opposed to, for example, the number of acoustic pulses needed to dispense that amount)(the energising chamber may, in some embodiments, be initially empty).

### DESCRIPTION OF THE DRAWINGS

Some preferred embodiments of the invention will now be described by way of example and with reference to the accompanying drawings, of which:
**Figure 1** is an exploded isometric view of a nasal medication delivery device according to an embodiment of the invention;
**Figure 2** is a side cross-sectional view of the supply unit;
**Figure 3** is a side view of the exterior of the supply unit;
**Figure 4** is an expanded side cross-sectional view showing detail of the supply unit;
**Figure 5** is an isometric view of the supply unit;
**Figure 6** is an opposite isometric view of the supply unit;
**Figure 7** is schematic part cross-sectional view, and exterior view, of the device illustrating angled placement of various parts;
**Figure 8** is a side cross-sectional view showing detail of the medicine supply unit of an alternative embodiment of the invention showing a tapered medicine energising chamber; and
**Figure 9** is a side cross-sectional view of the medicine supply unit of an alternative embodiment of the invention showing an energising chamber which incorporates a well.

### DETAILED DESCRIPTION

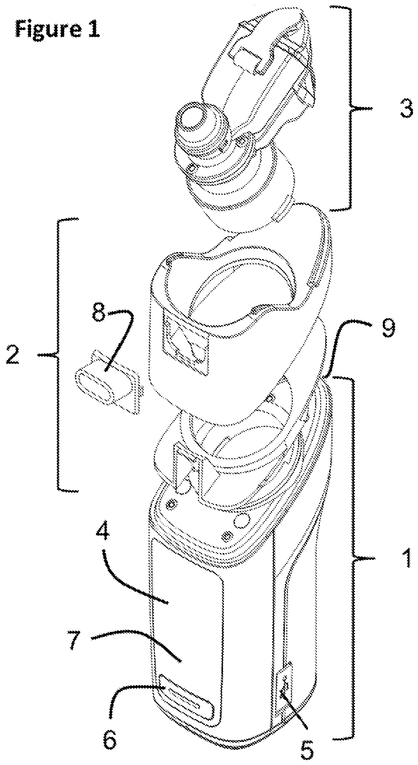

Referring to figure 1, the delivery device is used for the administration of therapeutic agents to the nasal cavity of a human patient. It has a main body 1, a breath receptor 2, and a medicine supply unit 3.

### The Main Body

The main body 1 has an LCD screen 4 to display messages to the user, for example the name of the medicine to be delivered, the time at which the next dose is due to be taken, etc. The main body 1 also has a battery, a USB port 5 for charging the battery or for receiving input generally, an on/off power switch 6, an RFID reader 7 and a breath sensor. All of the electronics for the delivery device are part of the main body 1.

The RFID reader 7 is able to read electronically stored prescription details kept on a tag on the medicine packaging. Those details are used to automatically set the device to deliver the prescribed amount of medicine.

### The Breath Receptor

The breath receptor 2 is detachable from the main body 1 with a click-fit arrangement, and has a detachable replaceable mouthpiece 8. The mouthpiece 8 can be gripped between the lips of the user and blown into. As the breath moves through the receptor 2 it is acoustically detected by the sensor in the main body 1, and, as a consequence, the device is triggered to deliver medicine to the user. The breath then passes out the back 9 of the device, having served its purpose. The device only releases medicine while the user is blowing/exhaling. This is because the act of exhaling causes the user's pharynx to close, so that medicine is only delivered to the nose, and not the throat or lungs.

### The Medicine Supply Unit

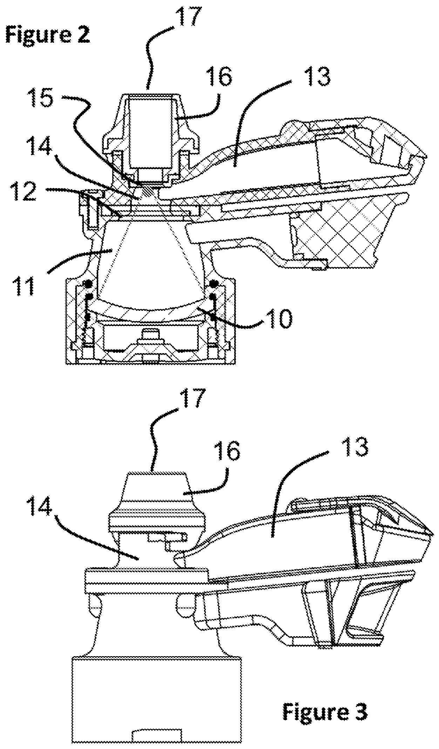

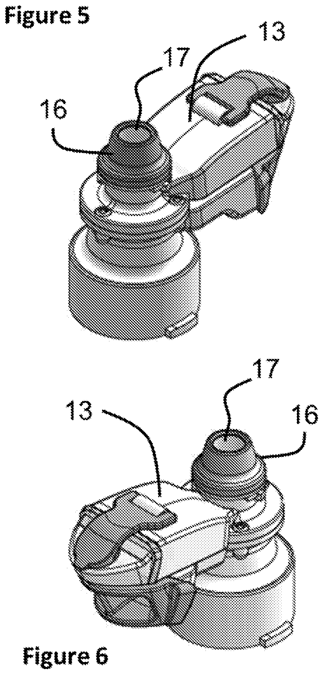

Referring to Figures 2 to 6, the medicine supply unit 3 has a concave piezoceramic ultrasonic transducer 10, ultrasonic energy transmission fluid 11 (optionally water), an ultrasonic transparent membrane 12 (which may be formed from a material such as polyetheretherketone (PEEK)), a medicine (liquid) feeder chamber 13, a liquid energising chamber 14, a mesh 15 and a nasal prong 16. The energising chamber 14 may or may not be asymmetrical or angled. The nasal prong 16 and a sub-assembly of the mesh engage the rest of the supply unit 3 by way of a twist lock mechanism to enable it to be easily cleaned or replaced.

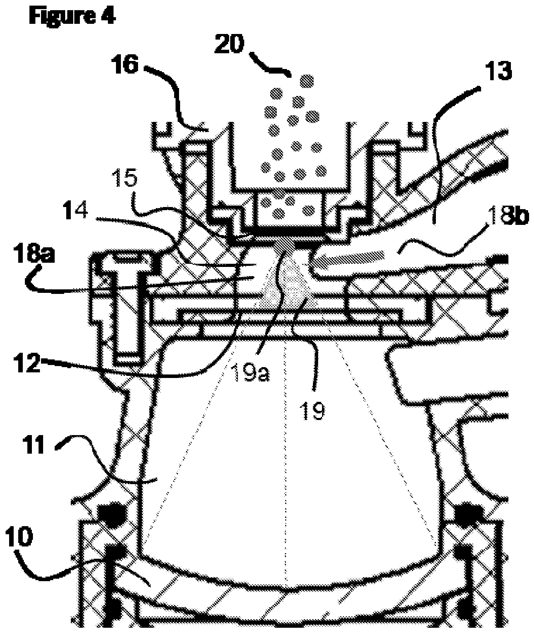

With particular reference to Figure 4, the transducer 10 is concave and emits ultrasonic energy that travels through the transmission fluid 11, through the membrane 12 and into a charge of medicine 18a in the energising chamber 14. The charge 18a becomes acoustically energized within an acoustic focal zone 19 created by the transducer, which produces an acoustic radiation pressure on the inner face of the mesh 15. As a result, the mesh 15 oscillates and produces a micro-pumping action of the medicine as it moves through the pores of the mesh 15. Such contact with the mesh 15 causes the medicine to become an aerosol 20, ie at the outer face of the mesh 15. The aerosol 20 then passes out of the nasal prong 16 via the exit 17. If the prong is held in the user's nose the aerosol enters his or her nasal cavity to give therapeutic relief.

With further reference to Figure 4, the concave nature of the transducer 10 concentrates ultrasonic energy at the focal zone 19 within the energising chamber 14 so that the charge of medicine 18a becomes acoustically activated. The dotted lines in Figure 4 illustrate an ultrasonic energy pathway resulting from the concavity of the transducer 10. The area referred to as the focal zone 19 is a small region of concentrated ultrasonic energy. As indicated, the focal zone preferably incorporates the focal point 19a, the position of which is dependent on the curvature of the transducer. In some embodiments of the invention the focal zone may be equivalent to the focal point.

In the preferred embodiment the energising chamber 14 is approximately 0.2 cm³ to 0.4 cm³ in size. The small size of the energising chamber 14 with respect to the liquid storage chamber 13 means that the size of the focal zone 19 is minimised.

This helps to minimise or substantially limit the amount of residual medicine (wastage) in the energising chamber 14 when the feeder chamber is exhausted.

The liquid medicine 18a in the energising chamber 14 is replenished with a new charge of medicine 18b under normal gravity from the feeder chamber 13. This ensures that there is always a charge of medicine 18a ready to be acoustically energized, that is until the medicine in the liquid storage chamber is emptied. When there is insufficient volume of medicine in the energising chamber 14 there will not be enough acoustic radiation pressure to effectively oscillate the mesh 15.

Referring to Figure 2, the mesh 15 is located within and is integral with the nasal prong 16, and the exit is an opening 17 at the end of the prong. The pore size of the mesh 15 determines, in part, the size of the aerosol droplets.

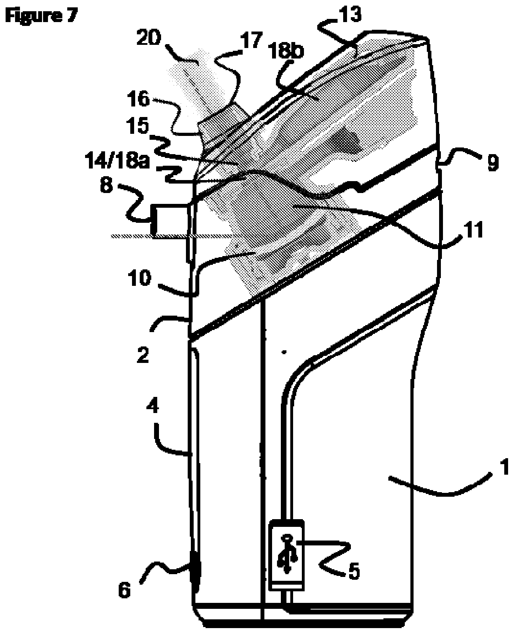

Referring to Figure 1, the medicine supply unit 3 can be readily detached from the main body 1 for cleaning, disinfection or replacement altogether. It connects to the body 1 via a cavity in the breath receptor 2. With particular reference to Figure 7, the medicine supply unit is angled slightly upwards to help the medicine flow more easily into the energising chamber 14 and to prevent bubbles forming in either the energising chamber or the transmission media 11. The angle also assists one to better align the path of aerosol leaving the mesh with targeted areas within the nasal cavity.

### Other Features

In some embodiments of the invention the device will only run if the user has purchased sufficient electronic 'credits', which are communicated to the device wirelessly or by hardwire.

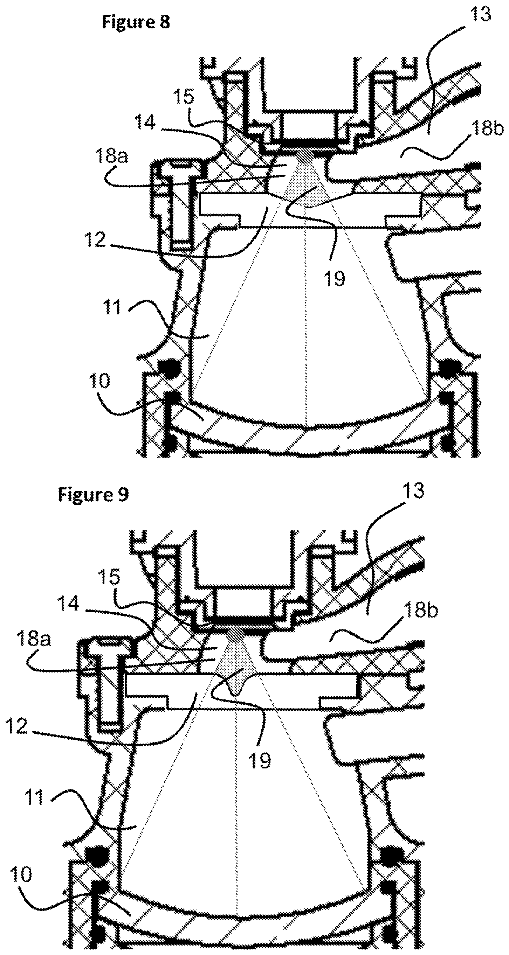

Referring to Figures 8 and 9, in some embodiments the energising chamber 13 has a tapered floor or a well provided by the membrane 12, designed to urge medicine 18a to gather centrally in the focal zone so as to prevent or reduce undelivered residues.

While some preferred forms of the invention have been described by way of example, it should be appreciated that modifications and improvements can occur without departing from the scope of the following claims.

## Claims

1. A device for delivering nasal medication, having:
- an ultrasonic energy generator, being a concave ultrasonic transducer, adapted to create an ultrasonic focal zone (19) of concentrated ultrasonic energy;
- a feeder chamber (13) holding medication;
- an energising chamber (14) smaller than the feeder chamber, arranged to hold medication at the focal zone (19);
- a mesh (15); and
- a nasal prong (16);
the device formed so that when it is activated:
- the feeder chamber (13) continuously fills the energising chamber (14) with medication, until the feeder chamber has insufficient medication left to achieve this, so that there is a substantially constant supply of medication within the focal zone (19) able to be energised and forced from the energising chamber (14) so as to contact the mesh (15), become an aerosol, and leave the device by way of the prong (16).

2. A device according to claim 1, having a mouthpiece (8) adapted for a human user to blow into and sensing means which, in response to such blowing, causes the medication to be energised and the aerosol to form.

3. A device according to claim 1 or 2, wherein the energising chamber (14) has a volume of 0.1 cm³ to 0.8 cm³.

4. A device according to any one of the preceding claims, wherein the ultrasonic generator is adapted to focus ultrasonic energy on the focal zone (19) when activated, such that medication in the energising chamber (14) becomes acoustically energised and moves towards the mesh (15).

5. A device according to any one of the preceding claims, formed such that it causes the mesh (15) to vibrate as the medication contacts the mesh (15) so as to assist in creating the aerosol.

6. A device according to any one of the preceding claims, having an exit (17) which is part of the prong (16).

7. A device according to claim 6, wherein the mesh (15) is integral with the prong (16).

8. A device according to claim 6 or 7, wherein the mesh (15) is immediately adjacent to the exit (17) so as to prevent or minimise condensation of the aerosol inside the device.

9. A device according to claim 6, 7 or 8, in each case when read as dependent on claim 2, wherein the energising chamber (14), the generator and exit (17) are at an angle of about 40° to about 80° with respect to the mouthpiece (8).

10. A device according to claim 6, 7 or 8, in each case when read as dependent on claim 2, or according to claim 9, wherein the energising chamber (14), the generator and exit are at an angle of about 50° to about 70° with respect to the mouthpiece (8).

11. A device according to any one of the preceding claims, wherein the medication is a pharmaceutical or saline solution.

12. A device according to any one of the preceding claims, wherein the feeder chamber (13) has a funnel or flume formed to naturally encourage the medication into the energizing chamber (14).

13. A device according to any one of the preceding claims, wherein the energising chamber (14) has:
a. a tapered floor and / or
b. a well;
to urge medication to gather at the focal zone (19).

14. A delivery device for nasal medication according to claim 1, wherein:
- the ultrasonic energy generator acoustically energises a charge of the medication within the focal zone to produce an acoustic radiation pressure on an inner face of the mesh so that the mesh oscillates to cause a micro-pumping action of the medication.

## Patentansprüche

1. Vorrichtung zum Abgeben eines Nasenmedikaments, die Folgendes aufweist:
- einen Ultraschallenergieerzeuger, der ein konkaver Ultraschallwandler ist, der angepasst ist, einen Ultraschallfokusbereich (19) aus konzentrierter Ultraschallenergie zu erzeugen;
- eine Zufuhrkammer (13), die ein Medikament aufnimmt;
- eine Erregungskammer (14), die kleiner als die Zufuhrkammer ist und eingerichtet ist, das Medikament in dem Fokusbereich (19) aufzunehmen;
- ein Netz (15); und
- eine Nasenspitze (16);
wobei die Vorrichtung geformt ist, so dass, wenn sie aktiviert ist:
- die Zufuhrkammer (13) die Erregungskammer (14) fortlaufend mit dem Medikament füllt, bis das Medikament in der Zufuhrkammer nicht mehr ausreicht, um dies zu erreichen, so dass ein im Wesentlichen konstanter Medikamentenvorrat innerhalb des Fokusbereichs (19) vorhanden ist, der angeregt und aus der Erregungskammer (14) gedrückt werden kann, um mit dem Netz (15) in Berührung zu kommen, um ein Aerosol zu werden und um die Vorrichtung über die Spitze (16) zu verlassen.

2. Vorrichtung nach Anspruch 1, die ein Mundstück (8) aufweist, das für einen menschlichen Benutzer angepasst ist, hineinzublasen, und Sensormittel, das als Reaktion auf ein solches Blasen bewirkt, dass das Medikament angeregt wird und das Aerosol ausgebildet wird.

3. Vorrichtung nach Anspruch 1 oder 2, wobei die Erregungskammer (14) ein Volumen von 0,1 cm³ bis 0,8 cm³ aufweist.

4. Vorrichtung nach einem der vorhergehenden Ansprüche, wobei der Ultraschallerzeuger angepasst ist, Ultraschallenergie auf den Fokusbereich (19) zu fokussieren, wenn er derart aktiviert wird, dass das Medikament in der Erregungskammer (14) akustisch erregt wird und sich auf das Netz (15) zubewegt.

5. Vorrichtung nach einem der vorhergehenden Ansprüche, die derart ausgebildet ist, dass sie das Netz (15) in Schwingungen versetzt, wenn das Medikament mit dem Netz (15) in Berührung gebracht wird, um ein Erzeugen des Aerosols zu unterstützen.

6. Vorrichtung nach einem der vorhergehenden Ansprüche, die einen Ausgang (17) aufweist, der Teil der Spitze (16) ist.

7. Vorrichtung nach Anspruch 6, wobei das Netz (15) einstückig mit der Spitze (16) ist.

8. Vorrichtung nach Anspruch 6 oder 7, wobei das Netz (15) unmittelbar an den Ausgang (17) angrenzt, um die Kondensation des Aerosols im Inneren der Vorrichtung zu verhindern oder zu minimieren.

9. Vorrichtung nach Anspruch 6, 7 oder 8, in jedem Fall, wenn sie in Abhängigkeit von Anspruch 2 gelesen wird, wobei die Erregungskammer (14), der Erzeuger und der Ausgang (17) in einem Winkel von etwa 40 ° bis etwa 80 ° hinsichtlich zu dem Mundstücks (8) angeordnet sind.

10. Vorrichtung nach Anspruch 6, 7 oder 8, in jedem Fall, wenn sie in Abhängigkeit von Anspruch 2 gelesen wird, in einem Winkel von etwa 50 ° bis etwa 70 ° hinsichtlich zu dem Mundstück (8) austreten.

11. Vorrichtung nach einem der vorhergehenden Ansprüche, wobei das Medikament eine pharmazeutische oder eine Salzlösung ist.

12. Vorrichtung nach einem der vorhergehenden Ansprüche, wobei die Zufuhrkammer (13) einen Trichter oder eine Rinne aufweist, der/die ausgebildet ist, um das Medikament auf natürliche Weise in die Erregungskammer (14) zu fördern.

13. Vorrichtung nach einem der vorhergehenden Ansprüche, wobei die Erregungskammer (14) Folgendes aufweist:
a. einen sich verjüngenden Boden und / oder
b. eine Vertiefung;
um das Medikament zu drängen, sich in dem Fokusbereich (19) zu sammeln.

14. Abgabevorrichtung für Nasenmedikamente nach Anspruch 1, wobei:
- der Ultraschallenergieerzeuger eine Ladung des Medikaments innerhalb des Fokusbereichs akustisch anregt, um einen akustischen Strahlungsdruck auf einer Innenoberfläche des Netzes zu erzeugen, so dass das Netz oszilliert, um eine Mikropumpwirkung des Medikaments zu bewirken.

## Revendications

1. Dispositif pour l'administration de médicament nasal, ayant :
- un générateur d'énergie ultrasonique, étant un transducteur ultrasonique concave, adapté pour créer une zone focale ultrasonique (19) d'énergie ultrasonique concentrée ;
- une chambre d'alimentation (13) contenant un médicament ;
- une chambre d'activation (14) plus petite que la chambre d'alimentation, agencée pour contenir le médicament au niveau de la zone focale (19) ;
- une maille (15) ; et
- une pince nasale (16) ;
le dispositif formé de telle sorte que lorsqu'il est activé :
- la chambre d'alimentation (13) remplit en continu la chambre d'activation (14) en médicament, jusqu'à ce que la chambre d'alimentation ne dispose plus suffisamment de médicament pour y parvenir, de telle sorte qu'il existe un approvisionnement substantiellement constant de médicament dans la zone focale (19) pouvant être activé et expulsé de la chambre d'activation (14) de manière à entrer en contact avec la maille (15), à devenir un aérosol et à quitter le dispositif par l'intermédiaire de la pince (16).

2. Dispositif selon la revendication 1, ayant un embout buccal (8) adapté à un utilisateur humain pour souffler dans celui-ci et des moyens de détection qui, en réponse à un tel soufflage, provoquent l'activation du médicament et la formation de l'aérosol.

3. Dispositif selon la revendication 1 ou 2, dans lequel la chambre d'activation (14) a un volume de 0,1 cm³ à 0,8 cm³.

4. Dispositif selon l'une quelconque des revendications précédentes, dans lequel le générateur ultrasonique est adapté pour concentrer l'énergie ultrasonique sur la zone focale (19) lorsqu'il est activé, de telle sorte que le médicament dans la chambre d'activation (14) devient acoustiquement activé et se déplace vers la maille (15).

5. Dispositif selon l'une quelconque des revendications précédentes, formé de telle sorte qu'il fait vibrer la maille (15) lorsque le médicament entre en contact avec la maille (15) de manière à aider à créer l'aérosol.

6. Dispositif selon l'une quelconque des revendications précédentes, ayant une sortie (17) qui fait partie de la pince (16).

7. Dispositif selon la revendication 6, dans lequel la maille (15) fait partie intégrante de la pince (16).

8. Dispositif selon la revendication 6 ou 7, dans lequel la maille (15) est immédiatement adjacente à la sortie (17) de manière à empêcher ou à minimiser la condensation de l'aérosol à l'intérieur du dispositif.

9. Dispositif selon la revendication 6, 7 ou 8, dans chaque cas, lorsqu'il est lu comme dépendant de la revendication 2, dans lequel la chambre d'activation (14), le générateur et la sortie (17) sont à un angle d'environ 40° à environ 80° par rapport à l'embout buccal (8).

10. Dispositif selon la revendication 6, 7 ou 8, dans chaque cas, lorsqu'il est lu comme dépendant de la revendication 2, la sortie est à un angle d'environ 50° à environ 70° par rapport à l'embout buccal (8).

11. Dispositif selon l'une quelconque des revendications précédentes, dans lequel le médicament est une solution pharmaceutique ou saline.

12. Dispositif selon l'une quelconque des revendications précédentes, dans lequel la chambre d'alimentation (13) a un entonnoir ou un canal formé pour encourager naturellement le médicament dans la chambre d'activation (14).

13. Dispositif selon l'une quelconque des revendications précédentes, dans lequel la chambre d'activation (14) a :
a. un fond conique et/ou
b. une cupule ;
pour inciter le médicament à se rassembler dans la zone focale (19).

14. Dispositif d'administration pour médicament nasal selon la revendication 1, dans lequel :
- le générateur d'énergie ultrasonique active acoustiquement une charge du médicament dans la zone focale pour produire une pression de rayonnement acoustique sur une face intérieure de la maille de sorte que la maille oscille pour provoquer une action de micro-pompage du médicament.
